# EUROPEAN PATENT APPLICATION

(11) **EP 4 628 159 A1**
(43) Date of publication of application: **08.10.2025**
(21) Application number: 23897121.2
(22) Date of filing: 19.07.2023
(51) Int. Cl.: A61N 5/10

(54) **DOSE ADJUSTMENT MECHANISM AND RADIATION THERAPY DEVICE**

(30) Priority: 01.12.2022 JP 2022193115
(71) Applicant: HITACHI HIGH-TECH CORPORATION, Tokyo 105-6409 (JP)
(72) Inventor: YOSHIDA, Koki, Tokyo 100-8280 (JP); TANIMOTO, Keiji, Tokyo 100-8280 (JP); ISHIZUKA, Takashi, Tokyo 100-8280 (JP); SAITOH, Yu, Tokyo 100-8280 (JP)
(74) Representative: MERH-IP Matias Erny Reichl Hoffmann Patentanwälte PartG mbB
(86) International application number: PCT/JP2023/026426
(87) International publication number: WO 2024/116461

(57) **Abstract**

A dose adjustment mechanism is to reduce an influence of radiation in the dose adjustment mechanism of a radiation therapy apparatus and to prevent occurrence of a failure. A dose adjustment mechanism 40 includes an actuator 42 disposed apart from a radiation axis irradiated with radiation by a radiation therapy apparatus 10, and a flattening filter 33 which is an interference element that moves in a direction perpendicular to the radiation axis by an operation of the actuator 42 and that is capable of switching between a state in which the interference element is disposed on an axis of the radiation axis and a state in which the interference element is not disposed on the axis of the radiation axis. An operation shaft of the actuator 42 does not intersect the radiation axis.

## Description

### Technical Field

The present invention relates to a dose adjustment mechanism and a radiation therapy apparatus.

### Background Art

The radiation therapy apparatus irradiates an affected area with radiation having directivity emitted from a target. In a plane perpendicular to a beam axis, a dose distribution of the radiation decreases as a distance from the beam axis increases, and has a mountain-like shape. Since it is difficult to make a therapy plan in the mountain-like shape, a flat dose distribution is obtained by a flattening filter (FF). Since the dose distribution varies depending on beam energy, the flattening filter is generally used depending on the beam energy.

The flattening filter is attached to the inside of an irradiation head. The flattening filter generally has a conical shape, and when a center of the cone deviates from the beam axis, a flatness of the dose at an isocenter greatly changes. Therefore, a position of the flattening filter needs to have highly accurate reproducibility.

In addition, in recent years, with the spread of highly accurate radiation therapy, such as intensity modulated radiation therapy and localization therapy, in which flattening of the dose distribution is not essential, therapy is performed in which irradiation is performed at a higher dose rate in a flattening filter-free (FFF) state without using a flattening filter.

Some existing radiation therapy apparatuses have a function of automatically switching the presence or absence of a flattening filter in the same apparatus (see, for example, PTL 1). The irradiation head is provided with a multi leaf collimator, a dose meter, and the like in addition to the flattening filter, and has a limited space. On the other hand, since the irradiation head is not so large because a patient and the apparatus easily interfere with each other, it is desirable to mount a mechanism for switching the presence or absence of the flattening filter in the limited space.

### Citation List

### Patent Literature

PTL 1: U.S. Patent No. 8890100

### Summary of Invention

### Technical Problem

When a structure such as an actuator is disposed near a beam axis as in the related art, there is a concern that a failure may occur due to an influence of radiation.

Therefore, an object of the invention is to provide a dose adjustment mechanism and a radiation therapy apparatus that reduce an influence of radiation and prevent occurrence of a failure.

### Solution to Problem

In order to achieve the above object, a typical dose adjustment mechanism of the invention includes: an actuator disposed apart from a radiation axis irradiated with radiation by a radiation therapy apparatus; and an interference element configured to move in a direction perpendicular to the radiation axis by an operation of the actuator and switch between a state in which the interference element is disposed on an axis of the radiation axis and a state in which the interference element is not disposed on the axis of the radiation axis. An operation shaft of the actuator does not intersect the radiation axis.

A typical radiation therapy apparatus of the invention includes: a radiation irradiation apparatus; a gantry configured to rotate the radiation irradiation apparatus around an isocenter; a patient support device configured to support a patient such that a therapy target site is positioned at the isocenter; and a dose adjustment mechanism configured to adjust a dose of radiation emitted by the radiation irradiation apparatus. The dose adjustment mechanism includes: an actuator disposed apart from a radiation axis irradiated with radiation by a radiation therapy apparatus; and an interference element configured to move in a direction perpendicular to the radiation axis by an operation of the actuator and switch between a state in which the interference element is disposed on an axis of the radiation axis and a state in which the interference element is not disposed on the axis of the radiation axis. An operation shaft of the actuator does not intersect the radiation axis.

### Advantageous Effects of Invention

According to the invention, it is possible to reduce an influence of radiation in a dose adjustment mechanism of a radiation therapy apparatus and prevent occurrence of a failure. Problems, configurations, and effects other than those described above will become apparent by the following description of embodiment.

### Brief Description of Drawings

[FIG. 1] FIG. 1 is a diagram of a radiation therapy apparatus.
[FIG. 2] FIG. 2 is a diagram of radiation irradiation (FF).
[FIG. 3] FIG. 3 is a diagram of radiation irradiation (FFF).
[FIG. 4] FIG. 4 is a diagram of a dose adjustment mechanism.
[FIG. 5] FIG. 5 is a diagram of the dose adjustment mechanism viewed from a direction of an electron gun.
[FIG. 6] FIG. 6 is a modification of the dose adjustment mechanism.
[FIG. 7] FIG. 7 is a diagram of control of the dose adjustment mechanism.

### Description of Embodiments

Hereinafter, an embodiment will be described with reference to the drawings.

### [Embodiment 1]

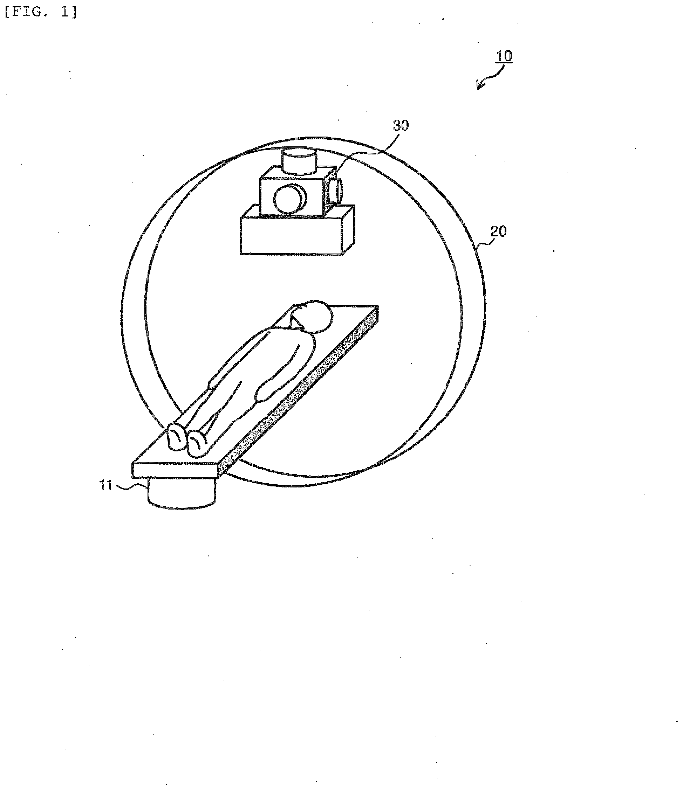

FIG. 1 is a diagram of a radiation therapy apparatus.

As shown in FIG. 1, the radiation therapy apparatus 10 includes a couch 11 as a patient support device, a gantry 20 including a rotation mechanism, and a radiation irradiation apparatus 30. The gantry 20 supports the radiation irradiation apparatus 30 and rotates the radiation irradiation apparatus 30 around the isocenter. The couch 11 disposes a therapy target site of a patient at the isocenter. The radiation irradiation apparatus 30 includes a head swinging mechanism and can swing an irradiation axis of radiation to be emitted.

The gantry 20 includes a ring frame and a rotation ring.

The ring frame is disposed such that a central axis thereof is oriented in a substantially horizontal direction. The rotation ring has an outer peripheral surface supported by an inner peripheral surface of the ring frame and is rotatable along the inner peripheral surface of the ring frame. The rotation ring is driven by a rotation drive mechanism and rotates around a rotation central axis.

The head swinging mechanism has a gimbal structure on which the radiation irradiation apparatus 30 is mounted, and can tilt the radiation irradiation apparatus 30 around two shafts of a pan shaft and a tilt shaft.

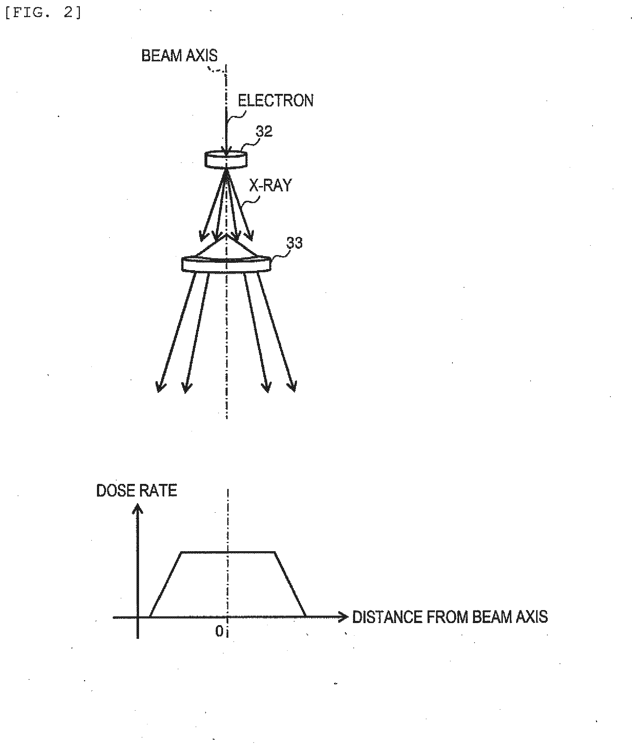

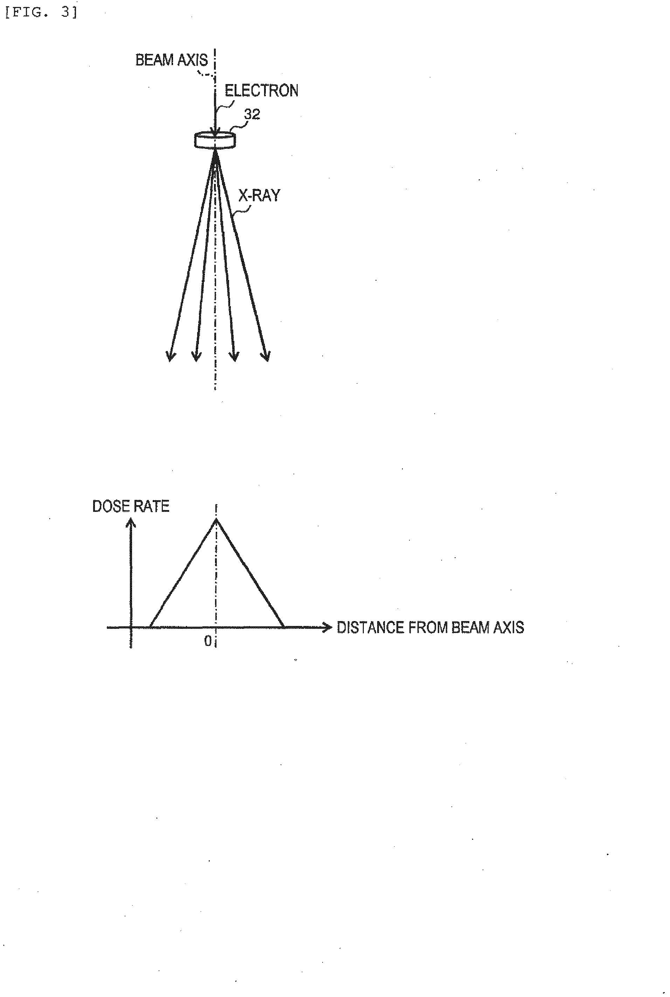

FIGS. 2 and 3 are diagrams of radiation irradiation. The radiation irradiation apparatus 30 accelerates electrons emitted from an electron gun to generate an electron beam, and irradiates a target 32 with the electron beam. The target 32 is irradiated with the electron beam and emits radiation (for example, X-rays).

A flattening filter 33 has a conical projection formed of aluminum and the like, and makes the dose distribution uniform in a plane perpendicular to a radiation direction of the radiation. FIG. 2 shows a relationship between a dose rate and a distance from a beam axis of the electron beam. As shown in FIG. 2, by applying the flattening filter 33, a value of the dose rate is the same in a certain range from the beam axis. A state in which the flattening filter 33 is applied is referred to as an FF state for convenience.

As shown in FIG. 3, in a flattening filter-free (FFF) state in which the flattening filter 33 is not used, the dose rate is highest on the beam axis and decreases according to the distance from the beam axis. Such irradiation in the FFF does not require flattening of the dose distribution such as intensity modulated radiation therapy and localization therapy, and is suitable for therapy requiring a higher dose rate.

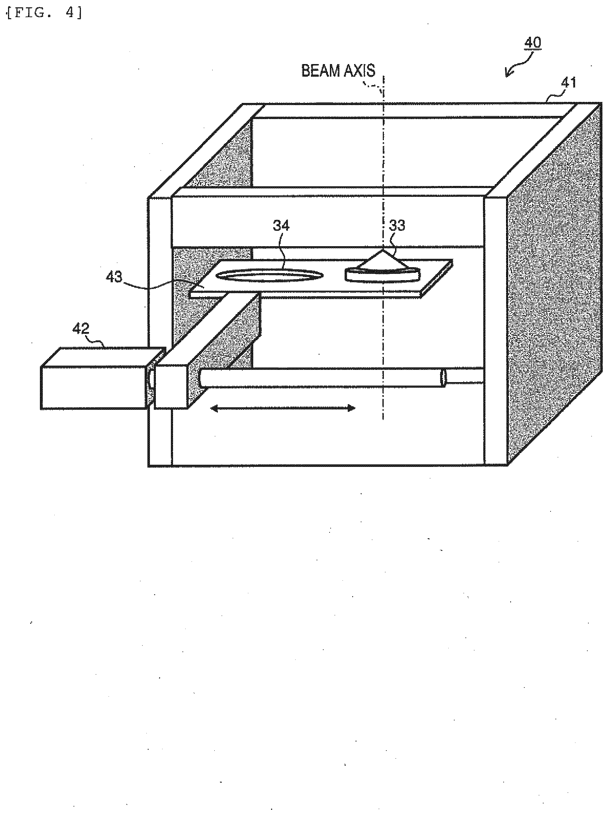

A dose adjustment mechanism 40 disclosed in the embodiment is a mechanism for switching between the FF and the FFF. FIG. 4 is a diagram of the dose adjustment mechanism.

The dose adjustment mechanism 40 includes a frame member 41, an actuator 42, a moving member 43, and a linear motion guide 44.

The frame member 41 is fixed with respect to a radiation axis (beam axis).

The actuator 42 is disposed on the outer side of the frame member 41.

The moving member 43 is a flattening filter arrangement portion on which the flattening filter 33 is mounted. The moving member 43 has a hole 34 through which the radiation is transmitted as it is.

The moving member 43 slides along the linear motion guide 44. That is, the linear motion guide 44 corresponds to a moving shaft of the moving member 43. The linear motion guide 44 implements a highly accurate linear operation of the moving member 43.

The moving member 43 is connected to the actuator 42 via a support member, and moves in a direction perpendicular to the radiation axis by an operation of the actuator 42. With this movement, the dose adjustment mechanism 40 switches between a state (FF) in which the flattening filter 33 is disposed on an axis of the radiation axis and a state (FFF) in which the hole 34 is positioned on the axis of the radiation axis.

The actuator 42 is disposed apart from the radiation axis in order to reduce an influence of radiation. An operation shaft of the actuator 42 is disposed not to intersect the radiation axis. Specifically, the operation shaft of the actuator 42 and a moving direction of the moving member 43 (a moving direction of the flattening filter 33) are parallel to each other, and the support member extends from the operation shaft of the actuator 42 and holds the moving member 43.

When the moving member is provided on the operation shaft of the actuator 42, the distance from the radiation axis to the actuator 42 increases, and a center of gravity moves away from the radiation axis. As the center of gravity is farther away from the radiation axis, there is a concern that the accuracy of the position control for the radiation irradiation may decrease. When the distance from the radiation axis to the actuator 42 is large, the possibility of interference with a gimbal mechanism increases.

On the other hand, as shown in FIG. 4, by disposing the operation shaft of the actuator 42 and a moving shaft of the flattening filter 33 in parallel, a position of the actuator 42 can be brought close to the radiation axis. As a result, the accuracy of radiation irradiation can be improved, and the interference with the gimbal mechanism can be reduced.

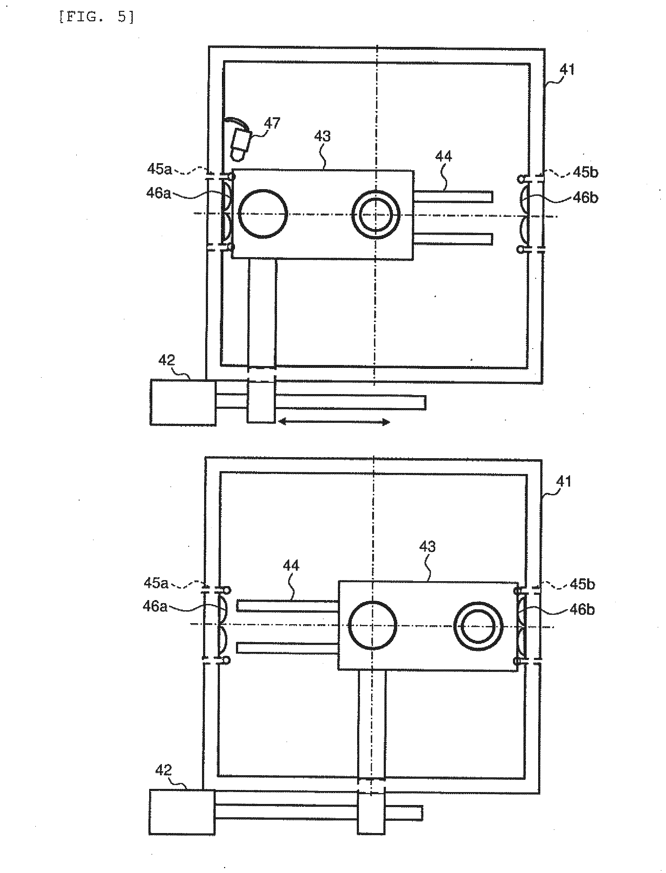

FIG. 5 is a diagram of the dose adjustment mechanism 40 viewed from the direction of the electron gun. The dose adjustment mechanism 40 is in the FF state in a state in which the moving member 43 is in contact with abutting stop surfaces 46a provided on an inner side of the frame member 41. In addition, the dose adjustment mechanism 40 is in the FFF state in a state in which the moving member 43 is in contact with abutting stop surfaces 46b facing the abutting stop surfaces 46a.

Abutting stop surfaces 46 (46a and 46b) have spherical surfaces and are in contact with the moving member 43 by the spherical surfaces. As an example, the abutting stop surface 46 may have a spherical segment shape cut out from a sphere. By forming the abutting stop surface 46 as a spherical surface, it is possible to reduce a probability of pinching a foreign substance.

As shown in FIG. 5, the dose adjustment mechanism 40 has the abutting stop surfaces 46 facing each other, and the actuator 42 presses the moving member 43 against any of the abutting stop surfaces 46 to switch between the two states. Therefore, position adjustment of the moving member 43 is unnecessary, and the state can be switched by a simple mechanism and control. As a result, the dose adjustment mechanism 40 can implement a small size and high accuracy at low cost.

The dose adjustment mechanism 40 includes sensors 45 in the vicinity of the abutting stop surfaces 46. Specifically, sensors 45a are provided in the vicinity of the abutting stop surfaces 46a, and sensors 45b are provided in the vicinity of the abutting stop surfaces 46b. The sensors 45 output the presence or absence of the moving member 43. The dose adjustment mechanism 40 can determine a consistency between the output of the sensors 45 and a control state of the actuator 42.

The dose adjustment mechanism 40 is in the FF state when the control state of the actuator 42 is a reference position. When an abnormality occurs in the operation of the actuator 42, the actuator 42 is likely to stop in a reference state. When the flattening filter 33 is turned on in the reference state, it is possible to improve safety when an abnormality occurs.

The dose adjustment mechanism 40 includes a nozzle 47 that injects air to the abutting stop surfaces 46 in order to remove foreign substance adhering to the abutting stop surfaces 46. The nozzle 47 may be provided for both the abutting stop surfaces 46a and the abutting stop surfaces 46b, but in the configuration of FIG. 5, the nozzle 47 is selectively provided for the abutting stop surfaces 46a. This is because the state in which the moving member 43 is in contact with the abutting stop surfaces 46a is the FF state and highly accurate positioning is required, whereas the state in which the moving member 43 is in contact with the abutting stop surfaces 46a is the FFF state and highly accurate positioning is not required.

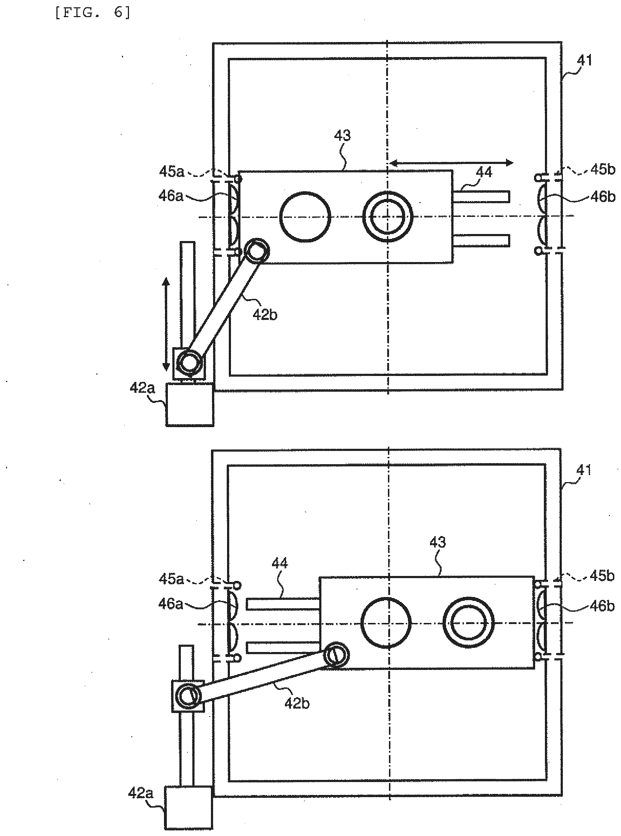

FIG. 6 is a modification of the dose adjustment mechanism 40. An operation shaft of an actuator 42a shown in FIG. 6 and the moving shaft of the moving member 43 are connected by a link member 42b, and a direction between the operation direction of the actuator 42 and the moving direction of the moving member 43 (the moving direction of the flattening filter 33) is changed. Since the actuator 42a positions the flattening filter 33 by continuously pressing the moving member 43, the accuracy can be ensured even when rattling occurs in a configuration using a plurality of components. Accordingly, with the configuration via the link member, a degree of freedom in arrangement of the actuator 42 is improved.

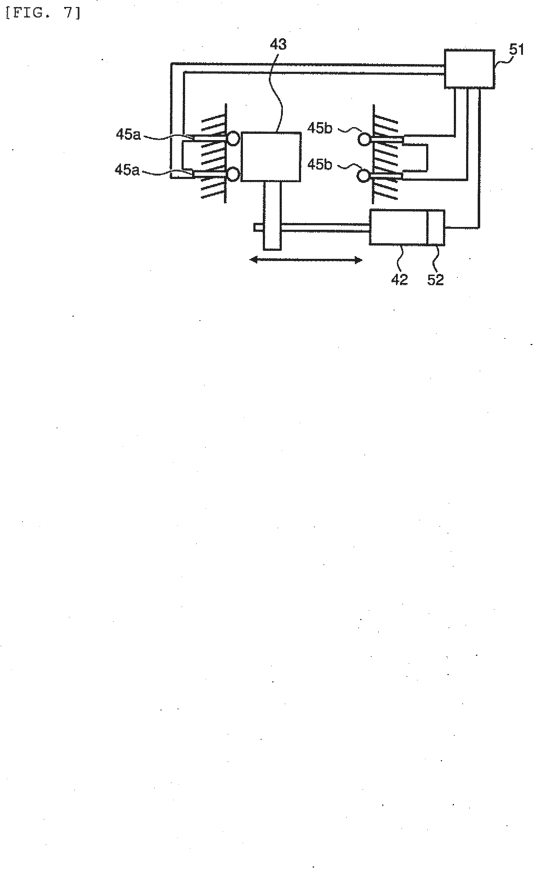

FIG. 7 is a diagram of control of the dose adjustment mechanism 40. The two sensors 45a are touch switches that are disposed in the vicinity of the abutting stop surfaces 46a and detect an FF mode. The two sensors 45b are touch switches that are disposed in the vicinity of the abutting stop surfaces 46b and detect an FFF mode.

The touch switches are connected in series and behave like an AND circuit. In FIG. 7, the two sensors 45a both detect contact, and a controller 51 detects the FF mode. A rough current position of the moving member 43 is monitored by an encoder 52.

Specifically, the dose adjustment mechanism 40 sequentially performs the following operations (1) to (5).
(1) A target position is transmitted from the controller 51 to the actuator 42 according to a selected mode, and the moving member 43 moves.
(2) When a value of the encoder 52 enters a certain range, a pressing operation starts. The pressing operation is an operation of continuing to move the moving member 43 toward the target position with a set force.
(3) When the moving member 43 is in contact with the abutting stop surface 46, the sensor 45, which is a touch switch, detects the contact, and the controller 51 detects a mode.
(4) Although the pressing operation is continued, since the position is not changed, a "pressing completion flag" is set in processing of the controller 51.
(5) Radiation irradiation is permitted by both of the detection of the mode and the "pressing completion flag".

As described above, the dose adjustment mechanism 40 of the disclosure includes the actuator 42 disposed apart from the radiation axis irradiated with radiation by the radiation therapy apparatus 10, and the flattening filter 33 which is an interference element that moves in the direction perpendicular to the radiation axis by the operation of the actuator 42 and that is capable of switching between the state in which the interference element is disposed on the axis of the radiation axis and the state in which the interference element is not disposed on the axis of the radiation axis. The operation shaft of the actuator 42 does not intersect the radiation axis.

As an example, the frame member 41 fixed with respect to the radiation axis may be further provided, the actuator 42 may be attached to a place in a stationary state with respect to the frame member 41, and the operation shaft may be configured to pass outside the irradiation range of the radiation.

Therefore, it is possible to reduce the influence of the radiation in the dose adjustment mechanism 40 of the radiation therapy apparatus 10 and to prevent occurrence of a failure.

Specifically, an actuator can be disposed away from a beam axis, and a failure probability due to radiation can be reduced.

In addition, since the actuator can be disposed outside a frame of an irradiation head, replacement and the like are easy, and maintainability is improved.

In addition, since a size of a mechanism in a filter moving direction can be reduced, the mechanism can be mounted in a limited space without increasing a size of an apparatus outer shape, and the reduction in a movable range of the apparatus can be prevented. It is possible to prevent enlargement of a region necessary for arrangement of the apparatus.

As an example, the operation shaft of the actuator 42 is parallel to the moving direction of the interference element, and the interference element is held via the support member extending from the operation shaft.

As an example, the direction between the operation direction of the actuator 42 and the moving direction of the interference element is changed via the link member 42b that connects the operation shaft of the actuator 42a and the moving shaft of the interference element.

The interference element is a filter that smooths an intensity distribution of the radiation.

The interference element may be an optical element. For example, a mirror that aligns an irradiation direction of light with a radiation axis may be used to illuminate a radiation irradiation field.

The dose adjustment mechanism 40 further includes the moving member 43 on which the interference element is mounted and which is moved by the operation of the actuator, and the frame member 41 fixed with respect to the radiation axis. The dose adjustment mechanism 40 is in the state (FF) in which the interference element is disposed on the axis of the radiation axis in a state in which the moving member 43 is in contact with a predetermined surface of the frame member 41.

Further, the dose adjustment mechanism 40 is in the state (FFF) in which the interference element is not disposed on the axis of the radiation axis in a state in which the moving member 43 is in contact with a surface facing the predetermined surface.

Accordingly, the dose adjustment mechanism 40 can easily switch between the FF and the FFF.

The dose adjustment mechanism 40 includes the sensor 45 in the vicinity of the predetermined surface, and determines the consistency between the output of the sensor 45 and the control state of the actuator 42.

In addition, in the dose adjustment mechanism 40, the interference element is disposed on the axis of the radiation axis when the control state of the actuator 42 is the reference position.

Therefore, it is possible to improve safety when an abnormality occurs.

The dose adjustment mechanism 40 further includes the nozzle 47 that injects air to the predetermined surface.

Therefore, foreign substance adhering to the predetermined surface can be removed, and a decrease in accuracy of positioning of the interference element can be avoided.

The invention is not limited to the above-described embodiment and includes various modifications. For example, the above-described embodiment has been described in detail to facilitate understanding of the invention, and the invention is not necessarily limited to those including all the configurations described above. In addition to deletion of such a configuration, it is also possible to replace or add a configuration.

For example, an element other than the flattening filter 33 may be used, or another element may be disposed in the hole 34.

### Reference Signs List

10: radiation therapy apparatus
11: couch
20: gantry
30: radiation irradiation apparatus
32: target
33: flattening filter
34: hole
40: dose adjustment mechanism
41: frame member
42: actuator
43: moving member
44: linear motion guide
45: sensor
46: abutting stop surface
51: controller
52: encoder

## Claims

1. A dose adjustment mechanism comprising:
an actuator disposed apart from a radiation axis irradiated with radiation by a radiation therapy apparatus; and
an interference element configured to move in a direction perpendicular to the radiation axis by an operation of the actuator and switch between a state in which the interference element is disposed on an axis of the radiation axis and a state in which the interference element is not disposed on the axis of the radiation axis, wherein
an operation shaft of the actuator does not intersect the radiation axis.

2. The dose adjustment mechanism according to claim 1, further comprising:
a frame member fixed with respect to the radiation axis, wherein
the actuator is attached to a place where the actuator is stationary with respect to the frame member, and
the operation shaft passes outside an irradiation range of the radiation.

3. The dose adjustment mechanism according to claim 1, wherein
the operation shaft of the actuator is parallel to a moving direction of the interference element, and the interference element is held via a support member extending from the operation shaft.

4. The dose adjustment mechanism according to claim 1, wherein
a direction between an operation direction of the actuator and a moving direction of the interference element is changed via a link member that connects the operation shaft of the actuator and a moving shaft of the interference element.

5. The dose adjustment mechanism according to claim 1, wherein
the interference element is a filter that smooths an intensity distribution of the radiation.

6. The dose adjustment mechanism according to claim 1, wherein
the interference element is an optical element.

7. The dose adjustment mechanism according to claim 1, further comprising:
a moving member on which the interference element is mounted and which is moved by the operation of the actuator; and
a frame member fixed with respect to the radiation axis, wherein
the interference element is disposed on the axis of the radiation axis in a state in which the moving member is in contact with a predetermined surface of the frame member.

8. The dose adjustment mechanism according to claim 7, wherein
the interference element is not disposed on the axis of the radiation axis in a state in which the moving member is in contact with a surface facing the predetermined surface.

9. The dose adjustment mechanism according to claim 7, further comprising:
a sensor provided in a vicinity of the predetermined surface, wherein
a consistency between an output of the sensor and a control state of the actuator is determined.

10. The dose adjustment mechanism according to claim 7, wherein
the interference element is disposed on the axis of the radiation axis when a control state of the actuator is a reference position.

11. The dose adjustment mechanism according to claim 7, further comprising:
a nozzle configured to inject air to the predetermined surface.

12. A radiation therapy apparatus comprising:
a radiation irradiation apparatus;
a gantry configured to rotate the radiation irradiation apparatus around an isocenter;
a patient support device configured to support a patient such that a therapy target site is positioned at the isocenter; and
a dose adjustment mechanism configured to adjust a dose of radiation emitted by the radiation irradiation apparatus, wherein
the dose adjustment mechanism includes
an actuator disposed apart from a radiation axis irradiated with radiation by a radiation therapy apparatus, and
an interference element configured to move in a direction perpendicular to the radiation axis by an operation of the actuator and switch between a state in which the interference element is disposed on an axis of the radiation axis and a state in which the interference element is not disposed on the axis of the radiation axis, and
an operation shaft of the actuator does not intersect the radiation axis.
